# EUROPEAN PATENT APPLICATION

(11) **EP 1 358 843 A1**
(43) Date of publication of application: **05.11.2003**
(21) Application number: 02711243.2
(22) Date of filing: 31.01.2002
(51) Int. Cl.: A61B 5/145

(54) **BIOLOGICAL INFORMATION PROCESSING SYSTEM, TERMINAL, BIOLOBICAL INFORMATION PROCESSOR, BIOLOBICAL INFORMATION PROCESSING METHOD, AND PROGRAM**

(30) Priority: 07.02.2001 JP 2001031427
(71) Applicant: MATSUSHITA ELECTRIC INDUSTRIAL CO., LTD., Kadoma-shi, Osaka 571-8501 (JP)
(72) Inventor: OOSHIMA, Kiyoko, Shijonawate-shi, Osaka 575-0024 (JP); UCHIDA, Shinji, Neyagawa-shi, Osaka 572-0807 (JP)
(74) Representative: Weigelt, Udo
(86) International application number: JP0200745
(87) International publication number: WO02062222

(57) **Abstract**

A biological information measuring system includes (1) a measuring device-side terminal 1, which has a measuring unit 2 of measuring biological information, an adapter unit 6 of obtaining a telephone number from a portable phone 7 to identify the portable phone 7, and transmitting and receiving means 11 of mutually linking the biological information and the telephone number and outputting the information, (2) the portable phone 7, which stores a telephone number to identify a user, and (3) a processing center 8, which has storing means 10 of storing a database in which the name of the user of the portable phone 7 or the like is linked with the telephone number, as well as of storing the biological information from the measuring device-side terminal 1 on a single user basis, and processing means 9 of identifying the user of the portable phone 7 on the basis of the telephone number and the database.

## Description

### Technical Field

The present invention relates to a biological information measuring system, a terminal unit, a biological information processing device, a biological information processing method and a program.

### Background Art

In recent years, as a system of supporting health care, home care or the like, a care system in which a user is connected to an information transmission center to transmit and receive information such as health condition has been under study.

Moreover, a survey conducted by the Ministry of Health and Welfare in Heisei year 9 (1997) to find out "the actualities of diabetes", revealed that the number of people who were strongly suspected of suffering from diabetes was 6 . 9 million, while including the people with a possibility of diabetes, the number reached as many as 13.7 million, which is disclosed in "2000 Seikatsusyukan-byo no Shiori" (Guide to Lifestyle-related diseases, Year 2000) published from Shakaihoken Syuppan-sha K. K. Inmost cases, the diabetes do not have a subjective symptom, and 45% of the people who are strongly suspected of suffering from diabetes are treated for the disease at present. Diabetes often becomes a cause of death due to its complication, and so diabetes is a very scary disease. However, for the reason that only a blood sugar level is high and no subjective symptom is developed in the early stage, it is not understood well enough that diabetes is a serious disease under the current circumstances. So, it is said that the most serious problem of the diabetes is that a diabetes patient misses a stage in which the patient can recover from the disease by a review of lifestyle and thereby becomes seriously ill.

In the diagnosis of diabetes, a fasting blood sugar level, a blood sugar level two hours after taking glucose or the like is checked. In the old days, to check a blood sugar condition at home, a patient carried out a test for urinary sugar on the patient's own. However, an exact blood sugar level cannot necessarily be checked only by the test for urinary sugar because there is a case where a blood sugar level is more than 200 mg although urinary sugar is negative, or to the contrary, there is a case where a blood sugar level is 100 mg or less although urinary sugar is strongly positive. Thus , there has been applied a method, in which an individual patient possesses a blood sugar measuring apparatus that can be used with simple operations, and the patient collects blood to measure a blood sugar level on the patient's own. Moreover, recently, there has been achieved a micro-invasive measuring device of measuring blood sugar level from an exudate on a body surface that is called humor, and the development of noninvasive measurement is also progressing.

Incidentally, the above mentioned care system is a system, in which a user transmits data obtained by individual measurement and the correspondence of inquiry is transmitted and received to obtain the provision of information and health care support. The aim of the care system is to establish telemedicine for home care, in which people can receive a doctor's guidance individually without going to the hospital. Accordingly, people who acknowledge that they are healthy do not take time to use this system, and so the system is used by only limited people.

Moreover, while it is important for patients seriously ill with diabetes to customarily measure blood sugar level because the value of blood sugar level affects the life, people who do not have a subjective symptom, however with a possibility of suffering from diabetes, or healthy people with no possibility of suffering from the disease, are not so interested in blood sugar level. Or, even though people have an interest in blood sugar level, they do not intend to possess a blood sugar measuring apparatus to customarily measure it.

In order that such people who do not have a subjective symptom know their blood sugar level, people should take medical examination in order to undergo a blood test, about once per year held by a company, for example, in the case of an office worker, and just voluntarily in the case of a self-employee, house wife or the like. Otherwise, it is difficult to know blood sugar level, and further, each individual does not conserve such data under the current circumstances.

Moreover, when people who want to know their blood sugar level voluntarily go to the hospital or clinic, there is a problem in that waiting time may be longer than time required for measurement, and this causes an obstacle for the frequent measurement of blood sugar level for people who are busy or live in a remote place. Thus, because people cannot check their blood sugar level easily, they further lose an interest in blood sugar level, and it can be said that this results in a delay in the finding of diabetes.

As stated above, it has previously been impossible for an indefinite number of people to easily measure biological information.

### Disclosure of the Invention

Thus, in view of the above problems, it is an object of the present invention to provide a biological information measuring system, which makes possible for an indefinite number of people such as those with a possibility of suffering from diabetes or those who are healthy but want to know their blood sugar level, to easily measure biological information such as blood sugar level without going to the hospital or clinic, and which also controls the measured biological information.

A 1st invention of the present invention (corresponding to Claim 1) is a biological information processing system comprising,
an information retaining device of retaining a certain information including identification information to identify a user,
a terminal unit having
(1) information obtaining means of obtaining said certain information from said information retaining device, and
(2) transmitting means of obtaining biological information of said user, relating the obtained biological information with said identification information, and transmitting it, and
a biological information processing device having
(1) receiving means of receiving said information transmitted from said terminal unit, and
(2) storing means of storing said biological information on the basis of said received identification information .

A 2nd invention of the present invention (corresponding to Claim 2) is the biological information processing system according to the 1st invention, wherein said terminal unit comprises biological information measuring means of measuring said biological information of said user.

A 3rd invention of the present invention (corresponding to Claim 3) is a terminal unit comprising,
information obtaining means of obtaining a certain information from an information retaining device of retaining said certain information including identification information to identify a user, and
transmitting means of obtaining biological information of said user, relating the obtained biological information with said identification information, and transmitting it to a biological information processing device.

A 4th invention of the present invention (corresponding to Claim 4) is an information retaining device of retaining a certain information including identification information to identify a user,
wherein said certain information is obtained by the terminal unit according to the 3rd invention.

A 5th invention of the present invention (corresponding to Claim 5) is a biological information processing device comprising,
receiving means of receiving information transmitted from a terminal unit, which has information obtaining means of obtaining a certain information from an information retaining device of retaining said certain information including identification information to identify a user, and transmitting means of obtaining biological information of said user, relating the obtained biological information with said identification information and transmitting it, and
storing means of storing said biological information on the basis of said received identification information.

A 6th invention of the present invention (corresponding to Claim 6) is the terminal unit according to the 3rd invention, wherein said certain information is obtained by said information obtaining means, using any one of Bluetooth, infrared radiation and wired communication system.

A 7th invention of the present invention (corresponding to Claim 7) is the biological information processing system according to the 1st invention, wherein said information retaining device is
(1) a portable telephone, in which said identification information is a telephone number of said portable phone, or
(2) a cash card, in which said identification information is a personal identification number of said cash card.

An 8th invention of the present invention (corresponding to Claim 8) is the information retaining device according to the 4th invention, wherein the device comprises measuring means having at least one function from among a step number counting function of counting step number data, a pulse wave measuring function of measuring pulse wave data, and a blood pressure measuring function of measuring blood pressure data, and
at least one from said step number data, said pulse wave data and said blood pressure data as well as said identification information are obtained by said terminal unit.

A 9th invention of the present invention (corresponding to Claim 9) is the biological information processing device according to the 5th invention, wherein said biological information processing device comprises:
advice information storing means of storing a plurality of advice information with respect to said biological information,
selecting means of selecting one or a plurality of advice information from among a plurality of advice information stored by said advice information storing means, on the basis of said biological information transmitted from said terminal unit, and
transmitting means of transmitting said advice information selected by said selecting means to said terminal unit or said information retaining device of said user.

A 10th invention of the present invention (corresponding to Claim 10) is the terminal unit according to the 3rd invention, wherein said terminal unit further comprises displaying and/or audio-outputting means of displaying and/or outputting by voice all or part of information from among said biological information measured by said biological information measuring means, said identification information, information regarding said user identified by said biological information processing device, and said biological information stored in said biological information processing device.

A 11th invention of the present invention (corresponding to Claim 11) is a biological information processing method comprising,
an information obtaining step of obtaining a certain information from an information retaining device of retaining said certain information including identification information to identify a user,
a transmitting step of obtaining biological information of said user, relating the obtained biological information with said identification information, and transmitting it from a terminal unit,
a receiving step of receiving said information transmitted from said terminal unit, and
a storing step of storing said biological information on the basis of said received identification information.

A 12th invention of the present invention (corresponding to Claim 12) is the biological information processing method according to the 11th invention, wherein said terminal unit is installed at a convenient store or in a place where an indefinite number of people gather.

A 13th invention of the present invention (corresponding to Claim 13) is a biological information transmitting method comprising,
an information obtaining step of obtaining a certain information from an information retaining device of retaining said certain information including identification information to identify a user, and
a transmitting step of obtaining biological information of said user, relating the obtained biological information with said identification information, and transmitting it to a biological information processing device.

A 14th invention of the present invention (corresponding to Claim 14) is a biological information processing method comprising,
a receiving step of receiving information transmitted from a terminal unit, which has information obtaining means of obtaining a certain information from an information retaining device of retaining said certain information including identification information to identify a user, and transmitting means of obtaining biological information of said user, relating the obtained biological information with said identification information and transmitting it, and
a storing step of storing said biological information on the basis of said received identification information.

A 15th invention of the present invention (corresponding to Claim 15) is a program allowing a computer to perform all or part of said information obtaining means, said transmitting means, said receiving means and said storing means of the biological information processing system according to the 1st invention.

A 16th invention of the present invention (corresponding to Claim 16) is a program allowing a computer to execute all or part of said information obtaining step, said transmitting step, said receiving step and said storing step of the biological information processing method according to the 11th invention.

A 17th invention of the present invention (corresponding to Claim 17) is a medium carrying the program according to the 15th invention and is computer processible.

A 18th invention of the present invention (corresponding to Claim 18) is a medium carrying the program according to the 16th invention and is computer processible.

### Brief Description of the Drawings

Figure 1 shows a configuration of a biological information measuring system in an embodiment of the present invention; and
Figure 2 shows a view of illustrating the flow of operations of a biological information measuring system in an embodiment of the present invention.

### (Explanation of Numerals)

- 1: Measuring device-side terminal
- 2: Measuring unit
- 3: Direct input unit
- 4: Display screen
- 5: Communication means
- 7: Portable phone
- 8: Processing center

### Best Mode for Carrying out the Invention

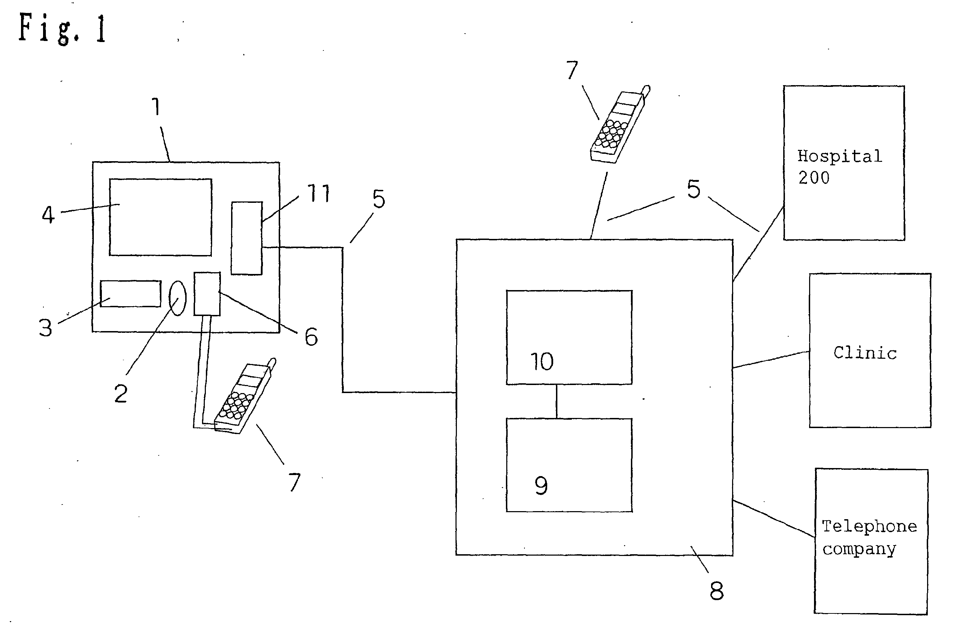

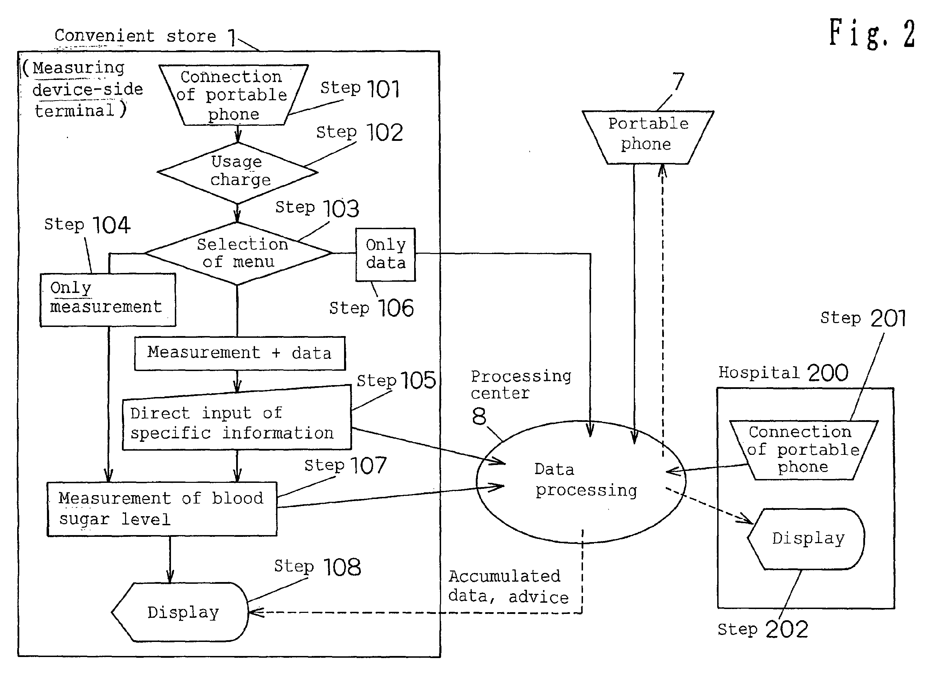

One embodiment of the present invention will be described below, using Figures 1 and 2. Herein, Figure 1 is a configuration view of a biological information measuring system in one embodiment of the biological information processing system of the present invention. Figure 2 is a block diagram showing the flow of the system.

In Figure 1, a measuring device-side terminal 1 is a terminal, which has a measuring unit 2 of optically measuring blood sugar level when a finger, for example, is placed on the measuring unit. This terminal 1 is installed in a place such as a 24 hours convenient store or a waiting room at the station or airport, where many people come and go, and which is open even at night. The measuring unit 2 is considered to consist of a known configuration.

This measuring device-side terminal 1 comprises a direct input unit 3 of inputting personal information such as date of birth, body weight and body height, and varied information such as time elapsed after eating when measurement is carried out, a display screen 4 of displaying service information such as advice obtained by analysis of measured data and other data, and transmitting and receiving means 11 of transmitting and receiving the measured data.

Moreover, inputting means in the direct input unit 3 may be a keyboard, or may be a touch panel configuration.

Furthermore, data transmitted and received by the transmitting and receiving means 11 is transmitted and received by a communication means 5 such as a general-purpose or special-purpose communication line.

Still further, in the measuring device-side terminal 1, an identification number specific for each device is stored as an ID, and when the transmitting and receiving means 11 transmits data to a controlling center 8, the above ID is also transmitted together with the transmitted and received data.

An adapter unit 6 is means of connecting and mounting a user side terminal 7 (hereinafter, referred to as a portable phone). In this adapter unit 6, there is embedded information obtaining means of reading information such as a telephone number or the like from the connected portable phone 7, and the read information is transmitted by the above communication means 5 to the processing center so as to identify a user.

It should be noted that information such as the telephone number of the above portable phone 7 is an example of the identification information of the present invention.

The processing center 8 is established specifically for the present system. The processing center 8 comprises processing means 9 of receiving, processing and returning data, and storing means 10 of storing the measured data or the like transmitted from the measuring device-side terminal 1.

Moreover, the processing center 8 is configured such that the center is constantly and mutually communicated with the above measuring device-side terminal 1, or e.g. a telephone company, a hospital 200 or a health care center by means of a general-purpose or special-purpose communication line.

The storing means 10 retains a database, in which the telephone number of the portable phone 7 is associated with attribute information such as the name of the user of the portable phone 7.

The processing means 9 is also means of identifying the user on the basis of the telephone number from the measuring device-side terminal 1 and the above database.

The information retaining device of the present invention corresponds to the portable phone 7 in the present embodiment. The transmitting means of the present invention corresponds to the communication means 5, and the terminal unit of the present invention corresponds to the measuring device-side terminal 1. The biological information measuring means of the present invention corresponds to the measuring unit 2. All of the receiving means, selecting means and transmitting means of the present invention correspond to the processing means 9. The biological information processing device of the present invention corresponds to a device comprising the processing means 9 and the storing means 10.

Next, while the operation and use of the biological information measuring system in the present embodiment will be explained, one embodiment of the biological information processing method of the present invention will also be explained simultaneously.

In the first measurement, when a user's own portable phone 7 is connected to the adapter unit 6 in the measuring device-side terminal 1 (refer to Step 101 in Figure 2), the telephone number is read from the connected portable phone 7. Then, the read information is transmitted by the transmitting and receiving means 11 to the above described processing center 8 so as to identify the user (no figures are shown).

Subsequently, there is displayed an item (refer to Step 102 in Figure 2) on a display screen 4, which is for the user to select whether the measuring system usage charge is included in the portable phone 7 charge and paid in a lump sum, or it is paid in cash on an as-needed basis. Then , the measuring system is started by operating a confirmation button in the direct input unit 3 (no figures are shown).

The measuring system is configured such that where "cash payment" is selected the system does not start until a designated fee is paid by throwing money into a slot (no figures are shown), which is established in the information measuring device-side terminal 1.

It should be noted that it is describe above that, when the portable phone 7 is connected to the adapter unit 6, the telephone number is read from the portable phone 7, but the adapter unit 6 may also be configured such that it obtains the telephone number from the portable phone 7 by using any one of Bluetooth, infrared radiation and wired communication system.

Next, the user inputs the user's own date of birth, body height, body weight and so on to the direct input unit 3 (refer to Step 105 in Figure 2) , and thereby specific advice can easily be provided depending on each user. This personal information may be input only for the first use.

After that, information such as time elapsed after eating is directly input to the direct input unit 3 (refer to Step 105 in Figure 2) , or it is selectively input using the display on the display screen 4 . These contents input are constantly displayed on the display screen 4 for confirmation, so that input errors are prevented.

Subsequently, by placing a finger on the measuring unit 2 and pressing a measurement starting button (no figures are shown) provided in the direct input unit 3, blood sugar level is measured and the data is displayed on the display screen 4. This measuring system may also be configured such that the measurement can automatically be started by placing a finger.

Moreover, as a body part used to measure blood sugar level, in addition to a finger, e.g. a palm, arm or earlobe may also be used.

Where the above described advice is not needed but only blood sugar level is needed (refer to Step 104 in Figure 2) , there can be made a selection, in which varied information such as, time elapsed after eating when measurement is performed, is omitted (refer to Step 103 in Figure 2).

The measured blood sugar level is automatically transmitted to the processing center 8 (Step 107 in Figure 2), and is then stored and accumulated in the storing means 10 on each user basis.

Where provision of advice is selected, for example, based on the above varied information, a message regarding determination level of diabetes, or advice on dietary instruction and exercise instruction that is determined from the age, body height and body weight of the user, is returned by the processing means 9 to the measuring device-side terminal 1 that is specified based on the above ID.

The thus returned message or the like and the measured blood sugar level are displayed on the display screen 4 (refer to Step 108 in Figure 2). It should be noted that these dietary and exercise instructions are out of the regulation of the Medical Practitioners Law or Pharmaceutical Affairs Law, since these instructions do not fall under treatment.

Where the provision of advice not needed is selected, only the measured blood sugar level is displayed on the display screen 4.

Also, the configuration is possible so that these displayed contents can be output by a printer function and so on. After completion, the portable phone 7 is removed.

From the second use, as with the case of the first use, the telephone number is read by connection of the portable phone 7, and the user is then confirmed in the processing center 8.

At that time, there is also an option which only refers to the previous data on the display screen 4 of the measuring device-side terminal 1 without measurement of blood sugar level (refer to Step 106 in Figure 2), and then a usage charge is presented depending on the content.

Moreover, personal information can simultaneously be confirmed whenever necessary. When body weight or the like is changed, the new data can be input, and the data can also be stored and fed back to advice depending on the new body weight.

The previously measured data is stored and accumulated by the storing means 10, and the measured data stored up till the last time is displayed on the display screen 4 in an easily readable graph form or the like (refer to Step 108 in Figure 2) . Regarding displayed contents , if measurement is carried out every after meal, alteration in a day can be observed. Otherwise, the contents can selectively be displayed whenever necessary in such a manner that the contents are accumulatively displayed every one week, every one month, etc., and thereby the blood sugar level of each individual can be controlled.

Moreover, the hospital 200 is connected to the processing center 8 online, and these accumulated measured data can be read out also from the hospital side.

Specifically, when a patient who is a user connects the patient's own portable phone 7 to the terminal on the hospital side (refer to Step 201 in Figure 2), the measurement data in the past time is displayed on the display screen of the terminal on the hospital side (refer to Step 202 in Figure 2).

Hereby, an interview with a doctor can be carried out smoothly on the basis of the displayed data.

Furthermore, in addition to time elapsed after eating, if simple selective input items regarding the menu of meals incepted or the like are established as varied information, a feedback can be carried out to provide further detailed advice.

It is naturally possible to select menu, for example, where it is burdensome to obtain advice on every measurement basis, it is possible to display only the measured value, and when the user has a sufficient time, the user can call up the accumulated data to obtain various advices (refer to Step 103 in Figure 2).

Still further, in the above described processing center 8, there is established a connection site from which the portable phone 7 captures the accumulated data, and it is also possible to display the data directly on the display screen on the portable phone 7, and so it is also possible to call up the accumulated data at home.

Thus, according to the present system, the measuring device-side terminal 1 is installed not only in a specific region, but also at a convenient store, which is open at many places in the country 24 hours a day (some stores are open from 7:00 to 23:00) 365 days a year, or a waiting room at the station or airport, which is open late at night, and it is therefore possible to perform measurement, while the user goes to a neighborhood or remote place.

Moreover, procedures at reception are required for measurement in the hospital, but by using the present system, such procedures become unnecessary. Accordingly, with a little spare time, measurement can easily be performed.

What is more, in the present system, since identification of the user is carried out using the portable phone 7 and data is automatically stored on every user basis, all what the user has to do is only to perform measurement.

Even if the user forgets the previously measured blood sugar level, there is no problem. That is to say, the user can obtain the previously accumulated data by connecting the portable phone 7 to the measuring device-side terminal 1 or a special site, and thereby health care comes to be extremely easily carried out.

Hence, since an individual person does not need to possess a special blood sugar measuring apparatus and further can perform measurement easily, the present system can be useful for health care of people who have no possibility of suffering from diabetes, children and so on.

Moreover, since the measuring device is installed in a familiar place, there are such effects that interest in blood sugar level is raised and people who have a possibility of suffering from diabetes are found in the early stage, etc.

Furthermore, it may also be possible that the portable phone 7 used in the present system is provided with a function of counting step number, which is obtained by known techniques and when the portable phone is connected to the adapter unit 6, the step number showing the distance the user walked, is read simultaneously to accumulate kinetic momentum. By this function, feedback on exercise advice can be provided in more detail, taking individual difference into consideration.

In a case where the user does exercise by playing sports though not having enough step number , accumulation of the kinetic momentum can be carried out by adopting a configuration, in which the type of sports, the time of playing sports and so on can selectively be input as personal information to the measuring device-side terminal 1.

Moreover, the portable phone 7 used in the present system may be provided with a function of measuring pulse wave obtained by known techniques.

For example, the portable phone may be equipped with a sensor unit comprising a light source and a photodetector. When the user places a finger on the sensor unit, light generated from the light source passes though the finger and propagates. Then, most of the light scatters, but some of the light reaches the photodetector. Since the reached light varies due to a blood flow rate, a pulse can be calculated by processing the amount varied.

When the portable phone 7 is connected to the adapter unit 6, the pulse data is also read simultaneously. Accordingly, a sign of the onset of arteriosclerosis or the like can be found by consecutively displaying the pulse data and analyzing the pulse wave shape.

It is difficult for the user to find the fluctuation of wave on the user's own, but the system can be so arranged that when there appears a disturbing anomaly, an advice to go to the hospital is displayed as a comment from the processing center 8.

However, this is not data obtained by consecutive measurement for 24 hours over many days , but data obtained by irregularly and intermittently measuring for a short time, and therefore it does not mean the sign of anomaly can be found without fail.

Moreover, if this pulse wave measuring function is applied to provide a function of simply measuring blood pressure, blood pressure is read simultaneously and feedback on appropriate exercise advice can also be provided.

Furthermore, the blood pressure measuring function may be provided in the above described measuring device-side terminal 1, and it may be configured such that the presence or absence of blood pressure measurement can be selected.

In the above embodiment, the portable phone 7 is used as an example of the measuring device-side terminal of the biological information measuring system of the present invention, but it may be configured such that a bank cash card, a credit card or a special card issued by the processing center 8 is used as a measuring device-side terminal.

At that time, a personal identification number or credit number are used as specific information instead of a telephone number. Moreover, at that time, to use such a card, a card slot is equipped in the measuring device-side terminal 1, and identification of the user can be carried out by inserting the card and inputting a personal identification number or the like.

By the use of a card, it becomes possible to measure blood sugar level and refer to accumulated data, even when the user is in areas where portable phones are inaccessible.

Moreover, in the above embodiment, information is displayed on the display screen 4, but for visually impaired people, information is not displayed on the display screen 4, but disclosure of data such as a measured value and provision of advice may be carried out by audio-outputting.

In such a case, the measuring method and measured data can also be informed separately by using a headphone. Thus, as with the above embodiment, menu for desired data such as only the measurement or provision of both the measured value and advice can be selected on an as-needed basis.

In the portable phone 7, an option may be provided to directly input the contents such as blood sugar level and advice, and thereby it is possible to replay them as a message from the portable phone 7.

As stated above, according to the present system which uses the user side terminal capable of identifying an individual person online, using the portable phone 7, a cash card or the like, the user can simply measure blood sugar level whenever the user wants without going to the hospital, clinic or health care center. Moreover, since data is automatically accumulated by connection of the user side terminal, even though the user forgets the data, the user can obtain information such as blood sugar level, a message and an advice by reconnection of the terminal, and thereby the user can carry out heath care with ease individually.

In the above embodiment, the terminal unit of the present invention is described as a configuration comprising the measuring unit 2 of measuring biological information, but it is not limited thereto. For example, the terminal unit may also be configured such that it does not comprise the measuring unit, but in stead, comprises means of obtaining the measured biological information from a portable phone in which biological information measuring means is embedded.

As a system configuration in this case, the biological information processing system comprises, for example, an information retaining device of retaining given information including identification information to identify a user; a terminal unit having (1) information obtaining means of obtaining the above given information from the above information retaining device, and (2) transmitting means of obtaining the biological information of the user, linking the obtained biological information with the above identification information and transmitting it; and a biological information processing device having (1) receiving means of receiving the above information transmitted from the above terminal unit, and (2) storing means of storing the above biological information on the basis of the above received identification information. Moreover, in this case, it may be configured that the biological information is obtained together with the identification information of the user by the information obtaining means of the present invention. According to such a configuration also, the same effects as stated above can be exerted.

Moreover, in the above embodiment, the present invention is described as a biological information processing system, but it is not limited thereto. For example, some devices constituting the present system also can constitute the present invention.

That is to say, the terminal unit of the present invention is a terminal unit, which comprises, for example, information obtaining means of obtaining given information from a information retaining device of retaining the above given information including identification information to identify a user, and transmitting means of obtaining the biological information of the above user, linking the obtained biological information with the above identification information and transmitting the information to a biological information processing device.

Furthermore, the information retaining device of the present invention is, for example, an information retaining device of retaining given information including identification information to identify a user, and the information retaining device is a device, wherein the above given information is obtained by the terminal unit according to claim 3.

Still further, the biological information processing device of the present invention comprises, for example, receiving means of receiving information transmitted from a terminal unit, which has information obtaining means of obtaining the given information from an information retaining device of retaining the above given information including identification information to identify a user and transmitting means of obtaining the biological information of the above user, linking the obtained biological information with the above identification information and transmitting the information, and storing means of storing the above biological information.

What is more, in the above embodiment, transmission of data from the processing center 8 is described regarding the case of using communication means such as those by wired or wireless communication, but the transmission is not limited thereto. For example, it may also be configured such that the measured data of the user accumulated in the processing center 8, a message, an advice and so on are informed to the user through the mail.

The present invention is a program, which allows a computer to execute functions of all or a part of the above described means (or devices, elements, circuits, parts etc.) of the biological information processing system of the present invention, and it is a program, which operates cooperatively with the computer.

Moreover, the present invention is a program, which allows a computer to execute operations of all or a part of the above described steps (or processes, operations, actions, etc.) of the biological information processing method of the present invention, and it is a program, which operates cooperatively with the computer.

Furthermore, the present invention is a medium, which carries a program allowing computer to execute functions of all or a part of the above described means of the biological information processing system of the present invention, and it is a medium which is computer-readable and the above read program executes the above functions cooperatively with the above computer.

Still further, the present invention is a medium, which carries a program allowing a computer to execute operations of all or a part of the above described steps of the biological information processing method of the present invention, and it is a medium which is computer-readable and the above read program executes the above operations cooperatively with the computer.

It shouldbe noted that a part of the means (or devices , elements, circuits, parts etc.) of the present invention or a part of the steps (or processes , operations, actions, etc.) of the present invention is herein used to mean several means or steps among such a plurality of means or steps, or a part of functions or operations in a means or step.

Moreover, apart of the devices ( or elements , circuits , parts etc.) of the present invention are herein used to mean some devices among such a plurality of devices, a part of means (or elements, circuits, parts, etc.) in a device, or a part of functions in a means.

Furthermore, ausage form of the program of the present invention may be an aspect, in which the program is recorded in a computer-readable recording medium and operates cooperatively with the computer.

Still further, a usage form of the program of the present invention may be an aspect, in which the program is transmitted in a transmitting medium and then read by a computer, and operates cooperatively with the computer.

What is more, examples of the recording medium include ROM or the like, and examples of the transmitting medium include a transmitting medium such as the Internet, a light, a radiowave, a sound wave, etc.

The above described computer of the present invention is not limited to pure hardware such as CPU, but may also include firmware, OS and peripheral equipment.

As stated above, the configuration of the present invention may be realized as software, or may be realized as hardware.

As is clear from the stated above, the present invention relates to a biological information measuring system, which supports the provision and control of biological information by measuring the biological information such as blood sugar level, and then transmitting and receiving the measured information. Therefore, the present invention can provide a biological information measuring system, in which an indefinite number of people can easily measure their own biological information and also control the measured biological information.

### Industrial Applicability

As is clear from the above explanation, the present invention has an advantage that an indefinite number of people can easily measure or control their own biological information.

## Claims

1. A biological information processing system comprising,
an information retaining device of retaining a certain information including identification information to identify a user,
a terminal unit having
(1) information obtaining means of obtaining said certain information from said information retaining device, and
(2) transmitting means of obtaining biological information of said user, relating the obtained biological information with said identification information, and transmitting it, and
a biological information processing device having
(1) receiving means of receiving said information transmitted from said terminal unit, and
(2) storing means of storing said biological information on the basis of said received identification information.

2. The biological information processing system according to claim 1, wherein said terminal unit comprises biological information measuring means of measuring said biological information of said user.

3. A terminal unit comprising,
information obtaining means of obtaining a certain information from an information retaining device of retaining said certain information including identification information to identify a user, and
transmitting means of obtaining biological information of said user, relating the obtained biological information with said identification information, and transmitting it to a biological information processing device.

4. An information retaining device of retaining a certain information including identification information to identify a user,
wherein said certain information is obtained by the terminal unit according to claim 3.

5. A biological information processing device comprising,
receiving means of receiving information transmitted from a terminal unit, which has information obtaining means of obtaining a certain information from an information retaining device of retaining said certain information including identification information to identify a user, and transmitting means of obtaining biological information of said user, relating the obtained biological information with said identification information and transmitting it, and
storing means of storing said biological information on the basis of said received identification information.

6. The terminal unit according to claim 3, wherein said certain information is obtained by said information obtaining means, using any one of Bluetooth, infrared radiation and wired communication system.

7. The biological information processing system according to claim 1, wherein said information retaining device is
(1) a portable telephone, in which said identification information is a telephone number of said portable phone, or
(2) a cash card, in which said identification information is a personal identification number of said cash card.

8. The information retaining device according to claim 4, wherein the device comprises measuring means having at least one function from among a step number counting function of counting step number data, a pulse wave measuring function of measuring pulse wave data, and a blood pressure measuring function of measuring blood pressure data, and
at least one from said step number data, said pulse wave data and said blood pressure data as well as said identification information are obtained by said terminal unit.

9. The biological information processing device according to claim 5 , wherein said biological information processing device comprises:
advice information storing means of storing a plurality of advice information with respect to said biological information,
selecting means of selecting one or a plurality of advice information from among a plurality of advice information stored by said advice information storing means, on the basis of said biological information transmitted from said terminal unit, and
transmitting means of transmitting said advice information selected by said selecting means to said terminal unit or said information retaining device of said user.

10. The terminal unit according to claim 3, wherein said terminal unit further comprises displaying and/or audio-outputting means of displaying and/or outputting by voice all or part of information from among said biological information measured by said biological information measuring means, said identification information, information regarding said user identified by said biological information processing device, and said biological information stored in said biological information processing device.

11. A biological information processing method comprising,
an information obtaining step of obtaining a certain information from an information retaining device of retaining said certain information including identification information to identify a user,
a transmitting step of obtaining biological information of said user, relating the obtained biological information with said identification information, and transmitting it from a terminal unit,
a receiving step of receiving said information transmitted from said terminal unit, and
a storing step of storing said biological information on the basis of said received identification information.

12. The biological information processing method according to claim 11, wherein said terminal unit is installed at a convenient store or in a place where an indefinite number of people gather.

13. A biological information transmitting method comprising,
an information obtaining step of obtaining a certain information from an information retaining device of retaining said certain information including identification information to identify a user, and
a transmitting step of obtaining biological information of said user, relating the obtained biological information with said identification information, and transmitting it to a biological information processing device.

14. A biological information processing method comprising,
a receiving step of receiving information transmitted from a terminal unit, which has information obtaining means of obtaining a certain information from an information retaining device of retaining said certain information including identification information to identify a user, and transmitting means of obtaining biological information of said user, relating the obtained biological information with said identification information and transmitting it, and
a storing step of storing said biological information on the basis of said received identification information.

15. A program allowing a computer to perform all or part of said information obtaining means, said transmitting means, said receiving means and said storing means of the biological information processing system according to claim 1.

16. A program allowing a computer to execute all or part of said information obtaining step, said transmitting step, said receiving step and said storing step of the biological information processing method according to claim 11.

17. A medium carrying the program according to claim 15 and is computer processible.

18. A medium carrying the program according to claim 16 and is computer processible.
